# EUROPEAN PATENT SPECIFICATION

(11) **EP 1 549 336 B1**
(45) Date of publication and mention of the grant of the patent: **05.01.2011**
(21) Application number: 03760322.2
(22) Date of filing: 13.06.2003
(51) Int. Cl.: A61K 38/22, A61K 35/74

(54) **PREVENTION AND/OR TREATMENT OF ULCERATIVE COLITIS USING PYY OR PYY[3-36]**
PRÄVENTION UND/ODER BEHANDLUNG VON COLITIS ULCEROSA MIT PYY ODER PYY[3-36]
PREVENTION ET/OU TRAITEMENT DE LA rectocolite hémorragique AU MOYEN DU PEPTIDE YY OU DU PEPTIDE YY [3-36]

(30) Priority: 14.06.2002 US 388930 P
(43) Date of publication of application: 06.07.2005
(73) Proprietor: AMYLIN PHARMACEUTICALS, INC., San Diego, California 92121 (US)
(72) Inventor: GEDULIN, Bronislava, Del Mar, CA 92014 (US); YOUNG, Andrew, A., La Jolla, CA 92037 (US); PATERNITI, James, R. Jr., San Diego, CA 92131 (US)
(74) Representative: Gowshall, Jonathan Vallance
(86) International application number: PCT/US2003/018657
(87) International publication number: WO 2003/105763

(56) References cited:
- IE-A1- 990 033
- US-A- 5 604 203
- LIU C D ET AL: "Intraluminal peptide YY induces colonic absorption in vivo." DISEASES OF THE COLON AND RECTUM APR 1997, vol. 40, no. 4, April 1997 (1997-04), pages 478-482, XP002457818 ISSN: 0012-3706
- LIU C D ET AL: "A novel synthetic analog of peptide YY, BIM-43004, given intraluminally, is proabsorptive." THE JOURNAL OF SURGICAL RESEARCH JUL 1995, vol. 59, no. 1, July 1995 (1995-07), pages 80-84, XP002457819 ISSN: 0022-4804
- EL-SALHY M. ET AL.: 'Peptide YY in gastrointestinal disorders' PEPTIDES vol. 23, February 2002, pages 397 - 402, XP002975289
- DATABASE MEDLINE [Online] DUMONT Y. ET AL.: 'Peptide YY derivatives as selective neuropeptide Y/peptide YY Y1 and Y2 agonists devoided of activity for the Y3 receptor sub-type', XP002975290 Retrieved from NCBI Database accession no. 7854062 & BRAIN RES. MOL. BRAIN RES. vol. 26, no. 1-2, October 1994, pages 320 - 324

## Description

### FIELD OF THE INVENTION

The field of the present invention relates to compositions and their use for treating ulcerative colitis.

### BACKGROUND OF THE INVENTION

Ulcerative colitis is a comparatively common inflammatory bowel disease ("IBD") with a prevalence of about 70-150 cases in a population of 100,000. There are estimated to be 380,000-480,000 persons in the United States with inflammatory bowel disease. Ward FM, et al., "Clinical economics review: medical management of inflammatory bowel disease." Aliment Pharmacol Ther. 1999;13(1):15-25. Ulcerative colitis typically exhibits a bimodal age distribution. For example, it usually appears in white males in their twenties and thirties and peaks at ages 20-29. In females, it peaks at about ages 30-39. Once affected, the disease is usually recurring with 75% of patients who suffered an initial attack continue to suffer repeated attacks. The second peaks for the disease in recurring patients is usually between the ages of 70-79 in both genders. Garland CF, et al., "Incidence rates of ulcerative colitis and Crohn's disease in fifteen areas of the United States." Gastroenterology. 1981;81(6):1115-24.

Although the clinical course of IBD is very variable, the disease can be debilitating and dangerous. It has been reported that less than 50% of patients survive 15 years and there is a 10-year mortality rate of 5-10%. The mortality risk of ulcerative colitis, for example, has been estimated to be about 1.7 times that of the general population. Gyde S, et al., "Mortality in ulcerative colitis." Gastroenterology. 1982;83(1 Pt 1):36-43. Surgical removal of the colon is eventually required in 20-25% of patients, and radical surgical intervention contributes substantially to the excess mortality in ulcerative colitis. Id., Cucino C. and Sonnenberg A., "Cause of death in patients with inflammatory bowel disease." inflamm Bowel Dis. 2001;7(3):250-5. An additional contributor to the increased risk of mortality is a propensity to develop colorectal cancer for patients who suffered from ulcerative colitis. The cumulative probability of such development of colorectal cancer has been estimated to be about 18% after 30 years of the disease. Eaden JA, et al., The risk of colorectal cancer in ulcerative colitis: a meta-analysis. Gut. 2001;48(4):526-35.

The major symptoms associated with local inflammatory bowel disease are bloody diarrhea and abdominal pain with blood- and pus-containing stools. The disease may also manifest systemically due to chronic malabsorption (e.g., dehydration, anemia, and hypokalemia) and inflammation (e.g. fever and weight loss). Systemic manifestations of the disease outside of the colon include arthritis, skin changes, and liver changes. Moreover, complications of the disease may include abscesses, fistulas, an increased risk of cancer, toxic megacolon (which carries a mortality of 50%), and bowel perforation with ensuing peritonitis and septicemia, (which is a significant cause of death). *Id*. Overall, for people affected with inflammatory bowel disease, the quality of life is markedly decreased.

Presently, the etiology of ulcerative colitis is unknown. Some have proposed and described a genetic predisposition to the disease, while others have proposed infective etiopathogenesis. There has also been reports of association between the onset of the disease with periods of emotional stress. Nevertheless, the conventional therapies for inflammatory bowel disease have focused on reducing inflammation of the colon. For example, present drug therapies for ulcerative colitis are either steroid-based (ACTH or glucocorticoids) or directed against prostaglandins such as 5-aminosalicylic acid (5-ASA) or sulfasalazine, which liberates 5-ASA after bacterial breakdown in the colon. But steroid therapies are often associated with major side effects including risk of infection and bone loss with the steroid doses required to control disease. Robinson RJ, et al., "Rectal steroids suppress bone formation in patients with colitis." Aliment Pharmacol Ther. 1997;11 (1):201-4.

Other experimental agents for the treatment of Crohn's disease, a form of inflammatory bowel disease, are similarly focused on suppressing the immune system of the patient suffering from the disease and may promote opportunistic infections. Some of these experimental drugs include (a) immunomodulators such as tacrolimus and mycophenolate; (b) biologics such as anti-tumor necrosis factor antibody, anti-interleukin-12 (IL-12) antibody, anti-alpha4 integrin antibody, interleukin-10 (IL-10), interleukin-11 (IL-11), and antisense RNA (ISIS-2302) against ICAM-1. In addition to promoting opportunistic infections, the immunomodulators are further limited by their toxicities at high doses or when used for long periods of time.

Other experimental drugs that are not anti-inflammatory have also been proposed but they are similarly limited by the major side effects associated with them. For example, thalidomide has also been studied as a drug for treating Crohn's disease, but the drug, originally released as a sedative and anti-nausea medication, was discontinued in the 1960s because it caused a high incidence of birth defects. Nicotine has also been used in clinical trials of ulcerative colitis and may work to inhibit production of interleukin-12 (IL-12) and tumor necrosis factor. However, randomized trials in active ulcerative colitis have shown only a modest benefit that is less than that of steroids, and it has relatively frequent side effects.

The alternative to the above therapies is surgery. Surgery and hospitalization, however, are estimated to be responsible for most of medical costs associated with inflammatory bowel disease, while drugs account for only about 10% of medical costs. Ward, *supra.* Furthermore, since ulcerative colitis, for example, affects people in the midst of their earning years, the indirect costs of the disease and incapacitation are disproportionately higher than in other diseases occurring later in life.

WO 03/026591 reports the use of PYY and PYY agonists for the modification of feeding behavior.

Thus, there is a major unmet medical need for treatments of inflammatory bowel disease including ulcerative colitis. Inflammatory bowel disease is comparatively common. It is chronic. It has a major impact upon the quality of life and upon the productivity of those affected, and few safe and effective treatments exist in the market today.

### SUMMARY OF THE INVENTION

The present invention provides a pharmaceutically active formulation of PYY or PYY agonist, wherein the PYY agonist is PYY[3-36], for use in the treatment of ulcerative colitis. The disclosure provides for the prevention, treatment or amelioration of subjects with inflammatory bowel disease, bowel atrophy, conditions characterized by loss of bowel mucosa or bowel mucosal function, and other conditions of the gastrointestinal tract, including those which may be brought about by exposure to cytotoxic agents, radiation, toxicity, infection and/or injury.

Thus, in a first aspect of the invention, treating inflammatory bowel disease is disclosed, and it, inter alia, comprises administering a pharmaceutically active formulation of PYY or PYY [3-36] to patients with ulcerative colitis. The administration of the PYY or PYY [3-36] may be achieved through any effective route of administration, preferably subcutaneous administration.

In a preferred embodiment of the invention, treatment of ulcerative colitis is provided by administering to a patient in need thereof a pharmaceutically active formulation of an effective PYY agonist, which is, PYY or PYY[3-36].

Use of PYY or PYY [3-36] may include formulating the PYY or the PYY [3-36] with a pharmaceutically accepted carrier and disseminating information on the administration of the PYY or PYY [3-36] to a patient with ulcerative colitis. The information may be disseminated through labeling of a drug product comprising the PYY or PYY [3-36] for the indicated use in ulcerative colitis characterized by loss of bowel mucosa or mucosal function, which may been brought about by exposure to cytotoxic agents, radiation, toxicity, or injury. Labeling may be achieved by any means recognized by the regulatory agency regulating the manufacture and distribution of drugs. For example, labeling may be achieved by a packaging insert for the drug product, affixing a paper containing the information onto a container containing the drug product, or by otherwise providing to health care professionals information, together or separate from the drug product, on the administration of PYY or PYY [3-36] to a patient with ulcerative colitis. Thus, a drug product comprising PYY or PYY [3-36] may be manufactured by formulating PYY or PYY [3-36] packaging the formulated PYY or PYY [3-36] and labeling the formulated PYY or PYY [3-36] for use in patients with the appropriate condition.

In another aspect of the invention, PYY or PYY [3-36] may also be administered in conjunction with an anti-inflammatory agent, or a growth hormone.

Herein disclosed is a method of manufacturing a PYY agonist by obtaining various compounds or PYY mutants and screening the compounds or mutants for binding to a PYY receptor. Any compound or mutant identified to be binding may then be administered to an animal that is a model for inflammatory bowel disease, bowel atrophy, or any other condition characterized by loss of bowel mucosa or bowel mucosal function. When the administration to the animal shows an ability of the compound or mutant to alleviate inflammatory bowel disease such as by reducing intestinal damage or inflammation in the colon, or by restoring bowel mucosa or bowel mucosal function, the compound and mutant may then be formulated with a pharmaceutically accepted carrier for administration in humans.

In another aspect of the invention, probiotic bacteria such as lactobacillus may be engineered to express PYY or PYY [3-36]. The probiotic bacteria comprising a nucleic acid sequence for the PYY or PYY [3-36] may then be introduced into the colon by any effective means including, for example, oral ingestion, suppository, or the like. Preferably, the lactobacillus culture expresses and secretes the PYY or PYY [3-36] in the colon.

### BRIEF DESCRIPTION OF THE DRAWINGS

FIGURE 1 demonstrates the ability of a PYY agonist, PYY[3-36] to reduce the colonic damage in an animal model for inflammatory bowel disease as compared to steroidal drugs.
FIGURE 2 depicts the effects on colon weight of a PYY agonist, PYY[3-36] as compared to steroidal drugs.
FIGURE 3 depicts the effects on spleen weight of a PYY agonist, PYY[3-36] as compared to steroidal drugs.

### GENERAL DESCRIPTION OF THE PREFERRED EMBODIMENT

As discussed above, conventional drug therapies for inflammatory bowel disease such as Crohn's disease focus on suppressing the immune system by using anti-inflammatory agents such as steroids, immunomodulators, and other biologics that affect inflammation. Contrary to these conventional drug therapies, it has been discovered and described herein that peripheral administration of PYY or agonists thereof protects the intestinal mucosa distal to the stomach and reduces the intestinal damage in the colon associated with inflammatory bowel disease without exerting a systemic immune suppressive effect. Such effect of PYY or a PYY agonist on the prevention and/or reduction of intestinal damage has not been previously demonstrated.

In one embodiment of the present invention, peripheral administration of an effective amount of PYY or PYY [3-36] protects the intestinal mucosa distal to the stomach. In another embodiment of the present invention, peripheral administration of an effective amount of PYY or PYY [3-36] reduces intestinal damage in the colon, including. In another embodiment, the effects of the invention may be accomplished while exerting a systemic immune suppressive effect less pronounced than with the use of steroid based anti-inflammatory compositions, and the like.

Peptide YY (PYY) is a polypeptide that is part of the pancreatic polypeptide (PP) family which include the pancreatic polypetide and neuropeptide Y (NPY). Pancreatic polypeptide was first discovered as a contaminant of insulin extracts and was named more by its organ of origin, rather than functional importance (Kimmel, Pollock et al. Endocrinology 83: 1323-30, 1968). It is a 36-amino acid peptide [SEQ ID NO.: 1] containing distinctive structural motifs. PYY was subsequently discovered in extracts of intestine and named Peptide YY (PYY) because of the N- and C- terminal tyrosihes (Tatemoto. Proc Natl Acad Sci U S A 79: 2514-8, 1982) [SEQ. ID NO.: 2]. A third peptide was later found in extracts of brain and named Neuropeptide Y (NPY) (Tatemoto. Proc Natl Acad Sci U S A 79: 5485-9, 1982; Tatemoto, Carlquist et al. Nature 296: 659-60, 1982) [SEQ ID NO.: 4].

Any effective PP ligand peptide or agonist may be used in accordance with the present disclosure.

The human sequences of peptides in the PP ligand family referred to herein are as follows (in conventional one-letter amino acid code):
PP: APLEPVYPGDNATPEQMAQYAADLRRYINMLTRPRY (SEQ ID NO: 1)
PYY: YPIKPEAPGEDASPEELNRYYASLRHYLNLVTRQRY (SEQ ID NO: 2)
PYY[3-36]: IKPEAPGEDASPEELNRYYASLRHYLNLVTRQRY (SEQ ID NO: 3)
NPY: YPSKPDNPGEDAPAEDMARYYSALRHYINLITRQRY (SEQ ID NO: 4)
These peptides are C-terminally amidated when expressed physiologically, but need not be for the purposes of the instant disclosure. These peptides may also have or lack other post-translational modifications.

PYY is released from endocrine cells in the distal ileum and colon. The release may occur, for example, in response to the presence of intraluminal lipids and carbohydrates and may control several gastrointestinal functions. Pappas TN, et al., "Enterogastrone-like effect of peptide YY is vagally mediated in the dog." J Clin Invest. 1986;77:49-53; Adrian TE, et al., "Effect of peptide YY on gastric, pancreatic, and biliary function in humans." Gastroenterology. " 1985;89:494-499. There has been evidence that indicates PYY's ability to modulate gastric motility through vagal dependent pathways and interact with PYY binding sites found in the area postrema and dorsovagal complex. Pappas, *supra;* Chen CH, et al., "PYY and NPY: control of gastric motility via action on Y1 and Y2 receptors in the DVC." Neurogastroenterol Motil. 1997;9:109-116. PYY has been shown to slow gastric emptying and small intestinal motility in various species, including rats, dogs, and humans. Al-Saffar et al., "Correlation between peptide YY-induced myoelectric activity and transit of small-intestinal contents in rats." Scand J Gastroenterol. 1985;20(5):577-82; Pappas, *supra;* Savage AP, et al., "Effects of peptide YY (PYY) on mouth to caecum intestinal transit time and on the rate of gastric emptying in healthy volunteers." Gut. 1987;28:166-70. It also reduces intestinal motility *in vitro,* and increases the transit time in conscious rats. Savage, *supra.* Exogenous PYY further increases water and electrolyte absorption in the intestine and colon of dogs. Liu CD, et al., "Intraluminal peptide YY induces colonic absorption in vivo." Dis Colon Rectum. 1997;40:478-82. The antisecretory effect of PYY in the rat jejunum appears to be mediated via Y2-receptor activation. Fu-Cheng X, et al., "Antisecretory effect of peptide YY through neural receptors in the rat jejunum in vitro." Peptides. 1999;20:987-93. In rats, vagal afferents may mediate a protective effect at the colon. Mazelin L, et al., "Protective role of vagal afferents in experimentally-induced colitis in rats." J Auton Nerv Syst. 1998;73:38-45.

In inflammatory bowel disease characterized by proximal malabsorption and steathorrea (such as tropical sprue characterized by small intestinal mucosal atrophy), plasma PYY concentrations have been reported to be elevated, perhaps due to stimulation by the inappropriate presence of fat in the distal bowel. Adrian TE, et al., "Peptide YY abnormalities in gastrointestinal diseases." Gastroenterology. 1986;90(2):379-84. However, in ulcerative colitis patients and in an IL-2 knockout mouse, which spontaneously develops ulcerative colitis, tissue and plasma PYY concentrations were reduced. *See* Tari A, et al. "Peptide YY abnormalities in patients with ulcerative colitis." Jpn J Med. 1988;27(1):49-55; Qian BF, et al., "Neuroendocrine changes in colon of mice with a disrupted IL-2 gene." Clin Exp Immunol. 2000;120(3):424-33. PYY administration prevented bowel mucosal protein loss during total parenteral nutrition and increased the weight and DNA content of the duodenum significantly in nursing rats and adult mice. However, others found no effect upon tissue mass and concluded that PYY is not trophic to the gastrointestinal tract. *See* Chance WT, et al., "Preservation of intestine protein by peptide YY during total parenteral nutrition." Life Sci. 1996;58(21):1785-94; Gomez G, et al., "Intestinal peptide YY: ontogeny of gene expression in rat bowel and trophic actions on rat and mouse bowel." Am J Physiol. 1995;268(1 Pt 1):G71-81; and Goodlad RA, et al., "Is peptide YY trophic to the intestinal epithelium of parenterally fed rats?" Digestion. 1990;46 Suppl 2:177-81.

As used herein, "PYY" is meant a Peptide YY polypeptide obtained or derived from any species. Thus, the term "PYY" includes both the human full length, 36 amino acid peptide as set forth in SEQ ID NO: 2, and species variations of PYY, including e.g., murine, hamster, chicken, bovine, rat, and dog PYY, for example. By "PYY agonist" is meant any compound which elicits an effect of PYY to protects from or reduce colon injury associated with inflammatory bowel disease or ulcerative colitis as exemplified in Example 1 described below and which binds specifically in a Y receptor assay (Example 4) or in a competitive binding assay with labeled PYY or PYY[3-36] from certain tissues having an abundance of Y receptors, including e.g., area postrema (Example 3), wherein the PYY agonist is not pancreatic polypeptide. Preferably, PYY agonists bind in such assays with an affinity of greater than 1 µM, and more preferably with an affinity of greater than 1-5 nM.

Such agonists can comprise a polypeptide having a functional PYY domain, an active fragment of PYY, or a chemical or small molecule. PYY agonists may be peptide or non-peptide compounds, and include "PYY agonist analogs," which refer to any compound structurally similar to a PYY that have PYY activity typically by virtue of binding to or otherwise directly or indirectly interacting with a PYY receptor or other receptor or receptors with which PYY itself may interact to elicit a biological response. Such compounds include derivatives of PYY, extended PYY molecules having more than 36 amino acids, truncated PYY molecules having less than 36 amino acids, and substituted PYY molecules having one or more different amino acids, or any combination of the above. Such compounds may also be modified by processes such as amidation, glycosylation, acylation, sulfation, phosphorylation, acetylation and cyclization.

One such PYY agonist analog is PYY[3-36], identified herein as SEQ ID NO: 3. Polypeptides with numbers in brackets refer to truncated polypeptides having the sequence of the full length peptide over the amino acid positions in the brackets. Thus, PYY[3-36] has a sequence identical to PYY over amino acids 3 to 36. A PYY agonist may bind to a PYY receptor with higher or lower affinity, demonstrate a longer or shorter half-life *in vivo* or *in vitro,* or be more or less effective than native PYY.

### Preparation and Formulation of PYY or PYY agonists

PYY and peptide-based PYY agonists described herein may be prepared in any effective manner, including, for example, standard recombinant expression, chemical peptide synthesis, and other techniques known in the art, e.g., using an automated or semiautomated peptide synthesizer. Solid phase peptide synthesis may be carried out with an automatic peptide synthesizer (e.g., Model 430A, Applied Biosystems Inc., Foster City, CA) using the NMP/HOBt (Option 1) system and tBoc or Fmoc chemistry (see, Applied Biosystems User's Manual for the ABI 430A Peptide Synthesizer, Version 1.3B July 1, 1988, section 6, pp. 49-70, Applied Biosystems, Inc., Foster City, CA) with capping. Peptides may also be assembled using an Advanced Chem Tech Synthesizer (Model MPS 350, Louisville, Kentucky). Peptides may be purified by any effective means, including, for example, RP-HPLC (preparative and analytical) using, e.g., a Waters Delta Prep 3000 system and a C4, C8 or C18 preparative column (10µ , 2.2 x 25 cm; Vydac, Hesperia, CA). Peptides may also be modified according to methods known in the art. For example, phosphate-containing amino acids and peptides containing such amino acids, may be prepared using methods known in the art. See, *e.g.,* Bartlett and Landen, Biorg. Chem. 14:356-377 (1986).

Peptide compounds useful in the disclosure may also be prepared using recombinant DNA techniques, using methods now known in the art. See, *e.g.,* Sambrook et al., Molecular Cloning: A Laboratory Manual, 2d Ed., Cold Spring Harbor (1989). Non-peptide compounds such as small molecule chemicals, carbohybrates, glycolipids useful in the present invention may also be prepared by methods known in the art or obtained from various sources that makes combinatorial libraries of non-peptide compounds.

Preferably, the PYY or PYY agonist is formulated with a pharmaceutically acceptable carrier for administration in humans. Various pharmaceutically acceptable carriers and their formulations are described in standard formulation treatises, e.g., Remington's Pharmaceutical Sciences by E.W. Martin. See also Wang, Y.J. and Hanson, M.A. "Parenteral Formulations of Proteins and Peptides: Stability and Stabilizers," Journal of Parenteral Science and Technology, Technical Report No. 10, Supp. 42:2S (1988).

The compositions or pharmaceutical composition described herein can be administered by any route, including intravenous, intraperitoneal, subcutaneous, and intramuscular, oral, rectal, topical, transmucosal, or by nasal and pulmonary inhalation. Compositions useful in the invention may conveniently be provided in the form of formulations suitable for parenteral (including intravenous, intramuscular and subcutaneous), nasal, oral, or rectal administration. In some cases, it will be convenient and desirable to provide a PYY or a PYY agonist and another agent such as an anti-inflammatory agent or growth hormone in a single composition or solution for administration together. In other cases, it may be more advantageous to administer the additional agent separately from said PYY or PYY agonist. A suitable administration format may best be determined by a medical practitioner for each patient individually.

Compounds useful in the disclosure can be provided as parenteral compositions for e.g., injection or infusion. Preferably, they are suspended in an aqueous carrier, for example, in an isotonic buffer solution at a pH of about 3.0 to about 8.0, preferably at a pH of about 3.5 to about 7.4, 3.5 to 6.0, or 3.5 to about 5.0. Useful buffers include sodium citrate-citric acid and sodium phosphate-phosphoric acid, and sodium acetate/acetic acid buffers. A form of repository or "depot" slow release preparation may be used so that therapeutically effective amounts of the preparation are delivered into the bloodstream over many hours or days following transdermal injection or delivery.

Since the products of the disclosure are amphoteric, they may be utilized as free bases, as acid addition salts or as metal salts. The salts must, of course, be pharmaceutically acceptable, and these will include metal salts, particularly alkali and alkaline earth metal salts, e,g., potassium or sodium salts. A wide variety of pharmaceutically acceptable acid addition salts are available. Such products are readily prepared by procedures well known to those skilled in the art.

For use by the physician, the compositions will be provided in dosage unit form containing an amount of a PYY or a PYY agonist with or without another active ingredient, e.g., an anti-inflammatory agent or a growth hormone. Therapeutically effective amounts of a PYY or a PYY agonist are those that alleviate inflammatory bowel disease, and other like conditions of the gastrointestinal tract. The compositions may, for example, reduce intestinal damage of the colon, reduce bloody diarrhea, or reduce abdominal pain, which may, for example, be associated with blood- and pus-containing stools. In another instance, the compositions restore bowel mucosa, or restore bowel mucosal function. These effects may also be obtained in combination. Generally, the effects may be accomplished while exerting a systemic immune suppressive effect less pronounced than with the use of steroid based anti-inflammatory compositions, and the like.

Assessment of damage in the colon may be performed using magnetic resonance imaging, ultrasound, radiography, video endoscopes, or any other suitable means under the direction of a physician or health care professional. *See e.g.,* "Madsen SM, et al. Inflammatory bowel disease evaluated by low-field magnetic resonance imaging. Comparison with endoscopy, 99mTc-HMPAO leucocyte scintigraphy, conventional radiography and surgery." Scand J Gastroenterol. 2002;37(3):307-16. As will be recognized by those in the field, an effective amount of therapeutic agent will vary with many factors including the age and weight of the patient, the patient's physical condition, the blood sugar level, the weight level to be obtained, and other factors

The exact dose to be administered is readily determined by one of skill in the art and is dependent upon the potency of the particular compound, as well as upon the age, weight and condition of the individual. The dosage of the compounds may range from about 100 µg to about 500 mg/ day, preferably about 500 µg to about 100 mg/day and more preferably about 1 mg to about 50 mg/day, for a 50 kg patient, administered in a single or divided doses. Administration may begin, for example, at the first sign of symptoms or shortly after diagnosis of inflammatory bowel disease or bowel atrophy. Administration may be by any route, e.g., injection, preferably subcutaneous or intramuscular, rectal, oral, nasal, transdermal, etc. Dosages for certain routes, for example oral administration, should be increased to account for decreased bioavailablity, for example, by about 5-100 fold.

The optimal formulation and mode of administration of compounds of the present application to a patient depend on factors known in the art such as the particular disease or disorder, the desired effect, and the type of patient. While the compounds will typically be used to treat human subjects they may also be used to treat similar or identical diseases in other vertebrates such as other primates, farm animals such as swine, cattle and poultry, and sports animals and pets such as horses, dogs and cats.

### Screening for Additional PYY Agonists

Other PYY agonists can be identified by using the receptor binding assays described below (e.g., in Examples 3 and 4) or known in the art in combination with the physiological screens described in the Examples below. Potential PYY agonists can be compared with the activity of PYY or PYY[3-36].

Alternatively, once one or more PYY-preferring (Y7) receptors have been characterized and cloned, alternative assays and high throughput screens can be implemented as discussed below or known in the art. Y7 receptors are those with an affinity for PYY or PYY[3-36] greater than their affinity for NPY. Methods of screening for compounds which modulate PYY receptor activity comprise contacting test compounds with PYY receptors and assaying for the presence of a complex between the compound and the PYY receptors. In such assays, the test ligand is typically labelled. After suitable incubation, free ligand is separated from that present in bound form, and the amount of free or uncomplexed label is a measure of the ability of the particular compound to bind to the PYY receptors. Alternatively, bound labelled ligand may be measured (e.g., using expressed membrane-bound Y7 receptors).

In another aspect of the disclosure, high throughput screening for compounds having suitable binding affinity to PYY receptors is employed. For example, large numbers of different small peptide test compounds are synthesised on a solid substrate. The peptide test compounds are contacted with the PYY receptor and washed. Bound PYY receptor is then detected by methods well known in the art. Purified test compounds can also be coated directly onto plates for use in the aforementioned drug screening techniques. In addition, if the test compounds are proteins, antibodies can be used to capture the protein and immobilize it on the solid support by any means known in the art.

Other apects of the disclosure comprise using competitive screening assays in which neutralizing antibodies capable of binding a polypeptide of the invention specifically compete with a test compound for binding to the polypeptide. In this manner, the antibodies can be used to detect the presence of any peptide that shares one or more antigenic determinants with a PYY agonist. Radiolabeled competitive binding studies are described in A.H. Lin et al. Antimicrobial Agents and Chemotherapy, 1997, vol. 41, no. 10. pp. 2127-2131.

Once a PYY agonist binding to the PYY receptor has been identified, the PYY agonist may be further assayed for its ability to protect intestinal mucosa or reduce damage of the colon, restore or protects bowel mucosa or mucosal function, according to the methods exemplified in Example 1. Finally, the PYY agonist may then be formulated with a pharmaceutically acceptable carrier for use in the administration to patients.

The PYY or PYY agonists may be provided as part of an article of manufacture that comprises a container containing the formulated PYY or PYY agonist. The article of manufacture may further comprise a label indicating the use of the formulated PYY or PYY agonist for patients with inflammatory bowel disease such as ulcerative colities and Crohn's disease. The label may be affixed to the container or may be a package insert provided with the article of manufacture.

As shown in the examples below, administration of PYY or an effective agonist of PYY in an animal model for inflammatory bowel disease significantly reduces intestinal damage of the colon. Accordingly, administration in humans is similarly beneficial in accordance with the present disclosure. The compositions may be used to prevent, treat and alleviate inflammatory bowel disease, bowel atrophy, or any other conditions characterized by loss of bowel mucosa or bowel mucosal function, including conditions that are brought about by exposure to cytotoxic agents, radiation, toxicity, infection and/or injury.

To assist in understanding the present disclosure, the following Examples are included.

### EXAMPLES

### Example 1: Reduction of Colon Injury of Animal Model for Inflammatory Bowel Disease Using PYY[3-36].

### Methods

*Animal preparation:* 10-11 weeks old male HSD rats, ranging 250-300 grams, were housed in a 12:12 light:dark cycle, and allowed *ad libitum* access to a standard rodent diet (Teklad LM 485, Madison, WI) and water. The animals were fasted for 24 hours before the experiment.

*Procedure:* A simple and reproducible rat model of chronic colonic inflammation has been previously described by Morris GP, et al., "Hapten-induced model of chronic inflammation and ulceration in the rat colon." Gastroenterology. 1989;96:795-803. It exhibits a relatively long duration of inflammation and ulceration, affording an opportunity to study the pathophysiology of colonic inflammatory disease in a specifically controlled fashion, and to evaluate new treatments potentially applicable to inflammatory bowel disease in humans.

Rats were anesthetized with 3% isofluorane and placed on a regulated heating pad set at 37°C. A gavage needle was inserted rectally into the colon 7 cm. The hapten trinitrobenzenesulfonic acid (TNBS) dissolved in 50% ethanol (v/v) was delivered into the lumen of the colon through the gavage needle at a dose of 30 mg/kg, in a total volume of 0.4-0.6 mL, as described in Mazelin, *supra.* Control groups received saline solution (NaCl 0.9%) intracolonically.

Four days after induction of colitis, the colon was resected from anesthetized rats, which were then euthanized by decapitation. Weights of excised colon and spleen were measured, and the colons photographed for scoring of gross morphologic damage according to Table 1 before division into 3 parts. The segment of colon 1 cm from the cecum was fixed in 10% formalin. Other segments were immediately frozen on dry ice.

Table 1 below provides the criteria for scoring gross morphological damage for this assay. Inflammation was defined as regions of hyperemia and bowel wall thickening. Colons were observed independently by several observers blinded to experimental treatment.

**Table 1.**

| **Score** | **Gross Morphology** |
|---|---|
| 0 | No damage |
| 1 | Localized hyperemia, but no ulcers |
| 2 | Linear ulcers with no significant inflammation. |
| 3 | Linear ulcer with inflammation at one site |
| 4 | Two or more sites of ulceration and/or inflammation |
| 5 | Two or more major sites of inflammation and ulceration or one major site of inflammation and ulceration extending >1 cm along the length of the colon |

For this assay, 5 treatment groups were set up as follows:
- Group A (n=3).: intracolonic saline with subcutaneous treatment of saline;
- Group B (n=8).: subcutaneous treatment of saline prior to induction of colitis by 30 mg/kg TNBS;
- Group C (n=10).: Experimental group -- subcutaneous treatment of PYY[3-36] at 900 µg/kg twice daily for 2 days before, 2 days after, and 30 min before induction of colitis by 30 mg/kg TNBS;
- Group D (n=9).: Positive control -- intramuscular injection of Depo-Medrol 600 µg/kg 60 min prior to induction of colitis by 30 mg/kg TNBS;
- Group E (n=10).: Positive control, 5-amino salicylic acid (Sigma) suspension at 200 mg/kg intracolonic administration in 0.5 mL saline, 60 min before induction of colitis and daily thereafter.

### Results

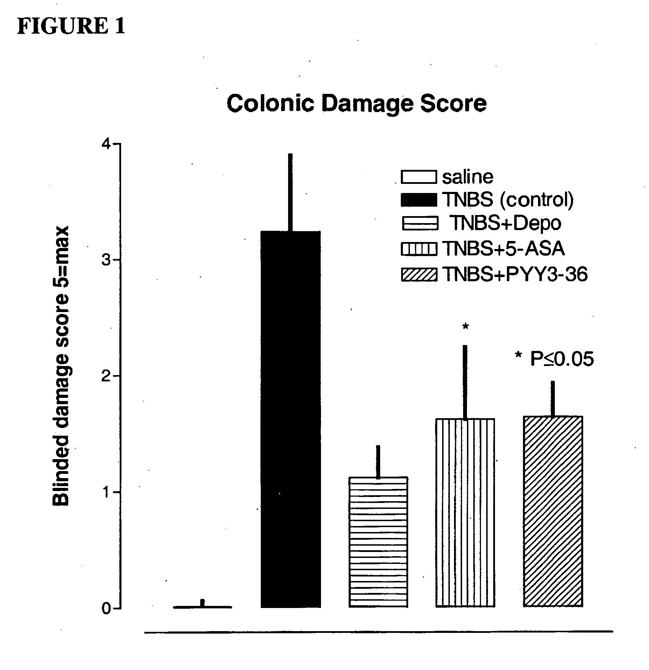

As seen in Figure 1, treatment of animals with 900µg/kg (twice daily) of PYY[3-36] before and after induction of colitis reduced the gross morphologic damage score index by about 50% in treated animals. Damage score index in animals treated subcutaneously with saline prior to induction of colitis was 3.24±0.66. In animals pretreated with PYY[3-36], the index was 1.64±0.29 (P=0.039). Steroid therapy (Depo-medrol) and 5-aminosalicilic acid (5-ASA) also reduced damage score index from 3.24±0.66 to 1.12±0.26 and 1.62±0.62 (P=0.02 and *P*=0.09, respectively vs saline).

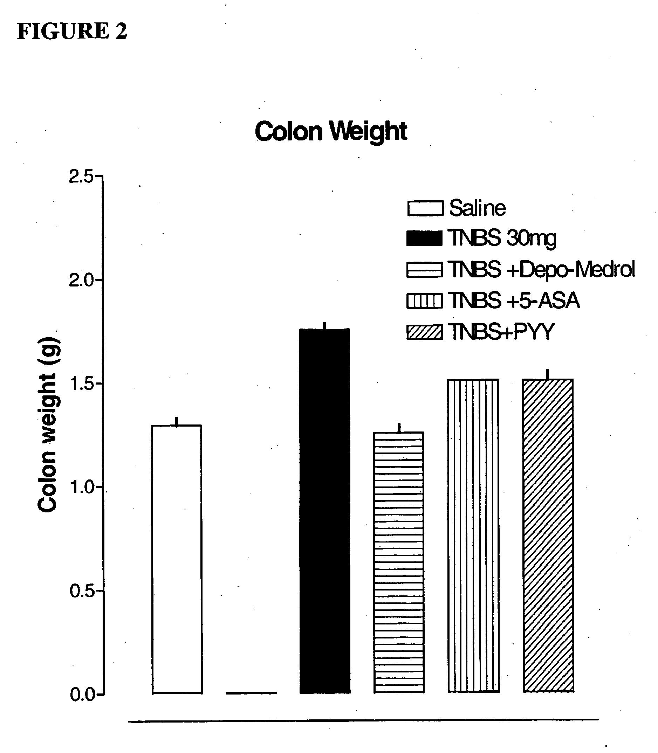

Figure 2 further provides evidence that PYY or a PYY agonist may be used to protect from colon injury. Figure 2 shows the experiment results based on the colon weight of the treated animals. Colon weight may somewhat reflect edema. Thus, increases in colon weight can be an indirect measure of injury. As seen in Figure 2, the pattern of colon weights mirrored that of the colon injury score in that increased in colon weight of PYY[3-36] treated rats were less than that of increase in colon weight of saline treatment after induction of colitis. Thus, these data demonstrate that administration of PYY or a PYY agonist to an animal may be used as an effective method treating inflammatory bowel disease including ulcerative colitis.

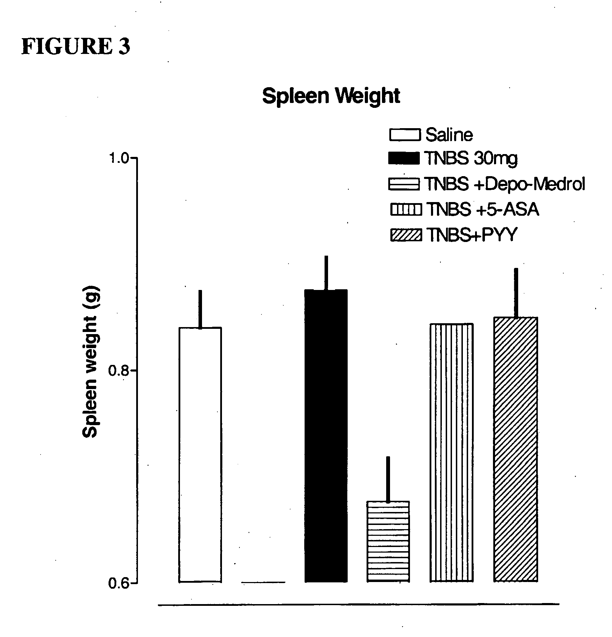

Immune suppression of PYY[3-36] treatment was also assayed by measuring the spleen weight of the treated animals. If there has been a systemic suppression of the immune system, the spleen weight of the animal will decrease as a result. As seen in Figure 3, the spleen weight in the Depo-Medrol positive control rats decreased and was consistent with a systemic immune suppression by Depo-Medrol. Spleen weight was not decreased in the 5-ASA positive control, which is consistent with the local action of 5-ASA. Spleen weight did not decrease in PYY-treated animals, suggesting that the effect of PYY[3-36] was not due to systemic immune suppression.

### Example 2: Other PYY agonist and analogs.

Although the example above used PYY[3-36], it is contemplated that other PYY agonists may also be used. Examples of other PYY agonists are disclosed in the literature, including U.S. Patent Nos. 5,604,203, 5,912,227, 5,916,869, 6,017,879, WO 03/026591.

Other PYY agonists may also be obtained by methods known in the art. For example, methods for determining peptide three-dimensional structure and analogs thereto are known, and are sometimes referred to as "rational drug design techniques." *See, e.g.,* U.S. Pat. No. 4,833,092 to Geysen; U.S. Pat. No. 4,859,765 to Nestor; U.S. Pat. No. 4,853,871 to Pantoliano; U.S. Pat. No. 4,863,857 to Blalock; see also Waldrop, Science 247, 28029 (1990); Rossmann, Nature 333, 392 (1988); Weis et al., Nature 333, 426 (1988); James et al., Science 260, 1937 (1993) (development of benzodiazepine peptidomimetic compounds based on the structure and function of tetrapeptide ligands).

In general, those skilled in the art will appreciate that deletion, substitution, or insertion may be made to the amino acid sequences of PYY to generate PYY mutants without unduly adversely affecting the activity thereof. Thus, peptides containing such deletions or substitutions are a further aspect of the present disclosure. For example, truncated mutants of PYY may be used according to the methods of the disclosure, where the truncation is a deletion of about 5, 10, 15, 25, or 30 amino acids from the n-terminus of PYY (e.g., PYY[6-36], PYY[11-36], PYY[16-36], PYY[26-36], and PYY[31-36]). In peptides containing substitutions or replacements of amino acids, one or more amino acids of a peptide sequence may be replaced by one or more other amino acids wherein such replacement does not affect the function of that sequence. Such changes can be guided by known similarities between amino acids in physical features such as charge density, hydrophobicity/hydrophilicity, size and configuration, so that amino acids are substituted with other amino acids having essentially the same functional properties. For example: Ala may be replaced with Val or Ser; Val may be replaced with Ala, Leu, Met, or Ile, preferably Ala or Leu; Leu may be replaced with Ala, Val or Ile, preferably Val or lie; Gly may be replaced with Pro or Cys, preferably Pro; Pro may be replaced with Gly, Cys, Ser, or Met, preferably Gly, Cys, or Ser; Cys may be replaced with Gly, Pro, Ser, or Met, preferably Pro or Met; Met may be replaced with Pro or Cys, preferably Cys; His may be replaced with Phe or Gln, preferably Phe; Phe may be replaced with His, Tyr, or Trp, preferably His or Tyr; Tyr may be replaced with His, Phe or Trp, preferably Phe or Trp; Trp may be replaced with Phe or Tyr, preferably Tyr; Asn may be replaced with Gln or Ser, preferably Gln; Gln may be replaced with His, Lys, Glu, Asn, or Ser, preferably Asn or Ser; Ser may be replaced with Gln, Thr, Pro, Cys or Ala; Thr may be replaced with Gln or Ser, preferably Ser; Lys may be replaced with Gln or Arg; Arg may be replaced with Lys, Asp or Glu, preferably Lys or Asp; Asp may be replaced with Lys, Arg, or Glu, preferably Arg or Glu; and Glu may be replaced with Arg or Asp, preferably Asp. Once made, changes can be routinely screened to determine their effects on function with assays exemplified in Examples 1, 3, and 4.

Non-peptide mimetics of PYY may also be used according to the method herein described. Non-protein drug design may be carried out using computer graphic modeling to design non-peptide, organic molecules able to bind to the PYY receptor. See, e.g., Knight, BIO/Technology 8, 105 (1990); Itzstein et al, Nature 363, 418 (1993) (peptidomimetic inhibitors of influenza virus enzyme, sialidase). Itzstein et al., Nature 363, 418 (1993), modeled the crystal structure of the sialidase receptor protein using data from x-ray crystallography studies and developed an inhibitor that would attach to active sites of the model; the use of nuclear magnetic resonance (NMR) data for modeling is also known in the art. See also Lam et al., Science 263, 380 (1994) regarding the rational design of bioavailable nonpeptide cyclic ureas that function as HIV protease inhibitors. Lam et al. used information from x-ray crystal structure studies of HIV protease inhibitor complexes to design nonpeptide inhibitors.

Agonists may also be developed by generating a library of molecules, selecting for those molecules which act as ligands for a specified target, in this case the PYY receptor, and identifying and amplifying the selected ligands. See, e.g., Kohl et al., Science 260, 1934 (1993) (synthesis and screening of tetrapeptides for inhibitors of farnesyl protein transferase, to inhibit ras oncoprotein dependent cell transformation). Techniques for constructing and screening combinatorial libraries of oligomeric biomolecules to identify those that specifically bind to a given receptor protein are known. Suitable oligomers include peptides, oligonucleotides, carbohydrates, nonoligonucleotides (e.g., phosphorothioate oligonucleotides; see Chem. and Engineering News, page 20, Feb. 7, 1994) and nonpeptide polymers (see, e.g., "peptoids" of Simon et al., Proc. Natl. Acad. Sci. USA 89, 9367 (1992)). See also U.S. Pat. No. 5,270,170 to Schatz; Scott and Smith, Science 249, 386-390 (1990); Devlin et al., Science 249, 404406 (1990); Edgington, BIO/Technology 11, 285 (1993). Peptide libraries may be synthesized on solid supports, or expressed on the surface of bacteriophage viruses (phage display libraries). Known screening methods may be used by those skilled in the art to screen combinatorial libraries to identify PYY receptor ligands. Techniques are known in the art for screening synthesized molecules to select those with the desired activity, and for labeling the members of the library so that selected active molecules may be identified. See, e.g., Brenner and Lerner, Proc. Natl. Acad. Sci. USA 89, 5381 (1992) (use of genetic tag to label molecules in a combinatorial library); PCT US93/06948 to Berger et al., (use of recombinant cell transformed with viral transactivating element to screen for potential antiviral molecules able to inhibit initiation of viral transcription); Simon et al., Proc. Natl. Acad. Sci. USA 89, 9367 (1992) (generation and screening of "peptoids", oligomeric N-substituted glycines, to identify ligands for biological receptors); U.S. Pat. No. 5,283,173 to Fields et al., (use of genetically altered Saccharomyces cerevisiae to screen peptides for interactions).

As used herein, "combinatorial library" refers to collections of diverse oligomeric biomolecules of differing sequence, which can be screened simultaneously for activity as a ligand for a particular target. Combinatorial libraries may also be referred to as "shape libraries", i.e., a population of randomized polymers which are potential ligands. The shape of a molecule refers to those features of a molecule that govern its interactions with other molecules, including Van der Waals, hydrophobic, electrostatic and dynamic.

Nucleic acid molecules may also act as ligands for receptor proteins. See, e.g., Edgington, BIO/Technology 11, 285 (1993). U.S. Pat. No. 5,270,163 to Gold and Tuerk describes a method for identifying nucleic acid ligands for a given target molecule by selecting from a library of RNA molecules with randomized sequences those molecules that bind specifically to the target molecule. A method for the in vitro selection of RNA molecules immunologically cross-reactive with a specific peptide is disclosed in Tsai, Kenan and Keene, Proc. Nati. Acad. Sci. USA 89, 8864 (1992) and Tsai and Keene, J. Immunology 150, 1137 (1993). In the method, an antiserum raised against a peptide is used to select RNA molecules from a library of RNA molecules; selected RNA molecules and the peptide compete for antibody binding, indicating that the RNA epitope functions as a specific inhibitor of the antibody-antigen interaction.

### Example 3: Area Postrema Assay For Identifying PYY agonist

Peripherally administered PYY has been reported to activate neurons in the area postrema (Bonaz, Taylor et al. Neurosci Lett 163: 77-80, 1993). Evaluation of the PYY agonist activity of potential compounds of the invention can be carried out using the *area postrema* assay as follows, in combination with an assay of PYY effect in protecting the intestines from damage according to the methods set forth in Example 1.

### Membrane Preparation

In this assay, *area postrema* membranes were prepared from tissue dissected from the pig or bovine brain stem. *Area postrema* membrane preparations are initiated by brief (4-10 seconds) homogenization of tissues using, e.g., a Polytron tissue homogonizer (Brinkman Instruments, NY) at ice-cold temperatures in a buffered solution such as phosphate buffered saline (138mM NaCl, 8.1mM Na₂PO_{4,} 2.5mM KCl, 1.2mM KH₂PO₄, 0.9mM CaCl₂, 0.5mM MgCl₂, pH 7.4). Following tissue disruption, large particles and debris were cleared by centrifugation (200 x g, 5 minutes, 4°C) and the supernatant fraction is preserved on ice. Membranes are isolated from the supernatant fraction by high-speed centrifugation (at least 40,000 x g, for at least 10 minutes, 4°C). Membranes are normally washed at least twice by re-homogenization in fresh buffer and recentrifugation, in order to remove endogenous interfering substances. Washed membranes are resuspended in buffer containing a proteolytic enzyme inhibitors such as phenylmethylsulfonyl fluoride (PMSF) or bacitracin. Volumes of buffer may be added sufficient to adjust the final tissue concentration to a level suitable for the particular screening method employed.

### Binding Reactions

In one embodiment, incubation mixtures for the screening method are set up as follows. To glass or polymeric tubes are added a small volume of Buffer Mixture ("HBBM") composed of a buffer solution such as HEPES containing a protease inhibitor such as bacitracin or PMSF, protease-free serum albumin (preferable fraction V BSA, protease-free) and, optionally, a Mg²⁺ or Ca²⁺ salt; and EDTA. To the Buffer Mixture is added a small volume of buffer containing the unlabeled molecules to be tested for agonist activity at concentrations of about 10⁻¹¹ to 10⁻⁶ M. Control tubes contain buffer alone. To this mixture is added amounts of labeled area postrema preparation ligand (here, PYY), in buffer so as to produce final concentrations of from about 10 to about 100 pM. Because of the high specific activities obtainable and ease of chemical labeling, ¹²⁵I is preferred to label the *area postrema* ligands. Ligands may be isolated from human tissues, from animal tissues, or produced by chemical, synthetic, or recombinant means. Labeled *area postrema* preparation ligands are dissolved in sterile water containing protease-free Fraction V BSA, aliquoted, and stored frozen until use.

Reactions are begun by adding, for example, membranes to each incubation tube. The amount of membrane protein required per tube is varied so that the amount of labeled ligand bound by the membranes in the assay is less than, for example, 10% of the total concentration of ligand in the assay (typically about 100 µg).

Reaction mixtures are incubated for a period of time and at a temperature sufficient to reach steady-state conditions within the period. The term "steady state" as used herein is intended to encompass the sum total of all reactions and processes that influence the net amount of bound hormone. It may or may not be synonymous with "equilibrium." Typically, tubes are incubated for about 60 minutes at room temperature.

### Detection

When membranes are used, they are isolated following binding in order to determine the amount of labeled ligand bound after competition between labeled and unlabeled ligands. It is convenient to collect membranes by filtration with a vacuum-powered Brandel Cell Harvester (Brandel Instruments, Gaithersburg, Maryland, Model M-24) through glass fiber filters (e.g., GF/B, Whatman) that have been presoaked with a regent in order to reduce nonspecific binding (NSB). Preferred is presoaking filters for about 5 hours in about 0.3% polyethyleneimine. The skilled artisan will know of other membrane collecting devices, such as the Millipore Filtration Assembly ((Model 1225) or the Sandbeck filter box (Bennett, J.P., in Neurotransmitter Receptor Binding, H.I. Yamura, et al., Raven, New York 1978, Pages 57-90), collecting filters, and NSB-reducing reagents that can be used in receptor binding assays. Both immediately before and immediately after filtration, filters are washed with large (milliliter) volumes of ice cold buffer to remove contaminating materials, e.g., unbound labeled ligand. Filters are removed and the amount of labeled ligand bound to membranes is quantified. Where ¹²⁵I is the label, radioactivity may be assessed in a gamma ray counter. Where a chemiluminescent reporter molecule (e.g., AMPPD, Tropix, Inc., Bedford, MA) is used, the light produced may be quantified in a luminometer. Enzymatic and fluorescent labels may also be used.

Instead of by filtration, membranes may be isolated following incubation by centrifugation (e.g., Beckman -2-21-M refrigerated centrifuge at 21,000 rpm or a Beckman 12 or Eppendorf microfuge), washed with ice cold buffer, then counted as such or following solubilization of membranes by detergent or alkali.

### Data Analysis

Scatchard plot saturation analyses of binding data, wherein bound/free (B/F) labeled ligand is plotted as a function of the amount bound, are performed by standard methods. See, e.g., (Scatchard. Ann NYAcad Sci 51: 660, 1949).

Competition curves, wherein the amount bound (B) is plotted as a function of the log of the concentration of ligand may be analyzed by computer, eg., analyses by nonlinear regression to a 4-parameter logistic equation (Prism Program; GraphPAD Software, San Diego, California) or the ALLFIT program (Version 2.7 (NIH, Bethesda, MD 20892)) (Munson and Rodbard. Anal Biochem 107: 220-39, 1980;de Lean, A., Munson, P. J. et al. 1988).

To determine binding constants, Scatchard saturation curves may be generated and analyzed according to a modification of the method of Scatchard, as described by Bylund, D.B., et al., "Methods for Receptor Binding," In H.I. Yamamura et al., eds., Methods in Neurotransmitter Analysis, Raven Press, New York, 1990 pp. 1-35.

In order to obtain specific binding values experimentally, a broad range of tracer concentrations of labeled ligand (typically, 1-150 pM) is used to obtain total binding and duplicate tubes reassessed, in the presence of a very high concentration, e.g., 100nM, of unlabeled ligand, to obtain nonspecific binding (NSM). The latter value is subtracted from each total binding value in order to obtain specific binding at every concentration of labeled ligand.

### Example 4: Y receptor binding assay for identifying PYY agonist.

Evaluation of the PYY agonist activity of potential compounds can be carried out by investigating their interaction with any of the known Y receptors, such as Y1-Y6, or with one or more unique receptor classes similar to the PYY-preferring receptors (such as Y7) expressed in cells, in combination with an assay of PYY effect, for example those of Examples 1 and 3. These cells may endogenously express the Y receptor of interest (such as SK-N-MC cells that express Y1 receptors or SK-N-BE2 cells which express Y2 receptors) or may be other cells (such as COS-7 or HEK293 cells) that are transfected with the clone of the Y receptor of interest. Binding to SK-N-BE2 cells is used as an example.

### Cell culture

SK-N-BE2 cells are grown on, for example 150 mm plates in tissue culture medium with supplements (Dulbecco's Modified Eagle Medium with 10% fetal calf serum, 4mM glutamine, 100 units/mL penicillin and 100µ/mL streptomycin) at 37°C in 5% CO₂ humidified atmosphere. Stock plates are trypsinized and split 1:6 every 3-4 days.

### Membrane Preparation

Cells are scraped from the plates in a small volume of a buffered solution such as phosphate buffered saline (138mM NaCl, 8.1mM Na₂PO₄, 2.5mM KCI, 1.2mM KH₂PO₄, 0.9mM CaCl₂, 0.5mM MgCl₂, pH 7.4), or are trypsinized, washed and resuspended in buffered solution. Membrane preparations are initiated by brief (10 seconds) homogenization of cells using, e.g., a Polytron tissue homogonizer (Brinkman Instruments, NY) at ice-cold temperatures. Membranes are further prepared by centrifugation as described above in Example 3. Binding reactions, detection and data analysis are as described in Example 3.

### Example 5: Probiotic bacteria expressing PYY or a PYY agonist

Probiotic bacteria that are present in the intestines may also exert a protective effect on the intestinal mucosal to prevent attack by other opportunistic bacteria on the intestional cells. Lactic acid bacteria are considered probiotic, and they may be used to express PYY or a PYY agonist to allow for increased protection. Among the lactic acid bacteria, the lactobacillus are preferred. For example, Lactobacillus rhamnosus GG (ATCC 53103), is a probiotic strain that has been isolated from healthy human intestinal flora. Its probiotic effects on human well being has been widely researched and documented in scientific journals.

Transformation of the lactobacillus may be performed using methods as described in Leer et al. (WO095/35389), and using vectors as described in PCT Application Nos. PCT/NL96/00409 and PCT/NL95/00135; U.S. Patent No. 6,100,388, and EP 1084709.

Cultures of lactobacillus expressing PYY or PYY agonists may be formulated as dry powder capsules or mixed into yogurt or milk. In a preferred embodiment, the PYY or PYY agonist is secreted by the lactobacillus in the intestines such that the PYY or PYY agonist can act to protect the intestinal mucosa cells.

Various modifications of the invention in addition to those shown and described herein will be apparent to those skilled in the art from the foregoing description.
1. Ward FM, Bodger K, Daly MJ; Heatley RV. Clinical economics review: medical management of inflammatory bowel disease. Aliment Pharmacol Ther. 1999;13(1):15-25.
2. Garland CF, Lilienfeld AM, Mendeloff AI, Markowitz JA, Terrell KB, Garland FC. Incidence rates of ulcerative colitis and Crohn's disease in fifteen areas of the United States. Gastroenterology. 1981;81(6):1115-24.
3. Gyde S, Prior P, Dew MJ, Saunders V, Waterhouse JA, Allan RN. Mortality in ulcerative colitis. Gastroenterology. 1982;83(1 Pt 1):36-43.
4. Cucino C, Sonnenberg A. Cause of death in patients with inflammatory bowel disease. Inflamm Bowel Dis. 2001;7(3):250-5.
5. Eaden JA, Abrams KR, Mayberry JF. The risk of colorectal cancer in ulcerative colitis: a meta-analysis. Gut. 2001;48(4):526-35.
6. Gordon FH, Hamilton MI, Donoghue S, et al. A pilot study of treatment of active ulcerative colitis with natalizumab, a humanized monoclonal antibody to alpha-4 integrin. Aliment Pharmacol Ther. 2002;16(4):699-705.
7. Robinson RJ, Iqbal SJ, Whitaker RP, Abrams K, Mayberry JF. Rectal steroids suppress bone formation in patients with colitis. Aliment Pharmacol Ther. 1997;11(1):201-4.
8. Hay JW, Hay AR. Inflammatory bowel disease: costs-of-illness. J Clin Gastroenterol.1992;14(4):309-17.
9. Walmsley RS, Ayres RC, Pounder RE, Allan RN. A simple clinical colitis activity index. Gut. 1998;43(1):29-32.
10. Madsen SM, Thomsen HS, Munkholm P, et al. Inflammatory bowel disease evaluated by low-field magnetic resonance imaging. Comparison with endoscopy, 99mTc-HMPAO leucocyte scintigraphy, conventional radiography and surgery. Scand J Gastroenterol. 2002;37(3):307-16.
11. Vucelic B, Milicic D, Krznaric Z, et al. [Serum acute phase proteins for determining disease activity of ulcerative colitis and Crohn disease]. Acta Med Austriaca. 1991;18(4):100-5.
12. Pappas TN, Debas HT, Taylor IL. Enterogastrone-like effect of peptide YY is vagally mediated in the dog. J Clin Invest. 1986;77:49-53.
13. Adrian TE, Savage AP, Sagor GR, et al. Effect of peptide YY on gastric, pancreatic, and biliary function in humans. Gastroenterology. 1985;89:494-499.
14. Chen CH, Stephens RL Jr, Rogers RC. PYY and NPY: control of gastric motility via action on Y1 and Y2 receptors in the DVC. Neurogastroenterol Motil. 1997;9:109-116.
15. Al-Saffar A, Hellstrom PM, Nylander G. Correlation between peptide YY-induced myoelectric activity and transit of small-intestinal contents in rats. Scand J Gastroenterol. 1985;20(5):577-82.
16. Pappas TN, Debas HT, Chang AM, Taylor IL. Peptide YY release by fatty acids is sufficient to inhibit gastric emptying in dogs. Gastroenterology. 1986;91:1386-9.
17. Savage AP, Adrian TE, Carolan G, Chatterjee VK, Bloom SR. Effects of peptide YY (PYY) on mouth to caecum intestinal transit time and on the rate of gastric emptying in healthy volunteers. Gut. 1987;28:166-70.
18. Liu CD, Newton TR, Zinner MJ, Ashley SW, McFadden DW. Intraluminal peptide YY induces colonic absorption in vivo. Dis Colon Rectum. 1997;40:478-82.
19. Fu-Cheng X, Souli A, Chariot J, Roze C. Antisecretory effect of peptide YY through neural receptors in the rat jejunum in vitro. Peptides. 1999;20:987-93.
20. Mazelin L, Theodorou V, More J, Fioramonti J, Bueno L. Protective role of vagal afferents in experimentally-induced colitis in rats. J Auton Nerv Syst. 1998;73:38-45.
21. Adrian TE, Savage AP, Bacarese-Hamilton AJ, Wolfe K, Besterman HS, Bloom SR. Peptide YY abnormalities in gastrointestinal diseases. Gastroenterology. 1986;90(2):379-84.
22. Tari A, Teshima H, Sumii K, et al. Peptide YY abnormalities in patients with ulcerative colitis. Jpn J Med.1988;27(1):49-55.
23. Qian BF, El-Salhy M, Melgar S, Hammarstrom ML, Danielsson A. Neuroendocrine changes in colon of mice with a disrupted IL-2 gene. Clin Exp Immunol. 2000;120(3):424-33.
24. Chance WT, Zhang X, Balasubramaniam A, Fischer JE. Preservation of intestine protein by peptide YY during total parenteral nutrition. Life Sci. 1996;58(21): 1785-94.
25. Gomez G, Zhang T, Rajaraman S, et al. Intestinal peptide YY: ontogeny of gene expression in rat bowel and trophic actions on rat and mouse bowel. Am J Physiol. 1995;268(1 Pt 1):G71-81.
26. Goodlad RA, Ghatei MA, Domin J, Bloom SR, Wright NA. Is peptide YY trophic to the intestinal epithelium of parenterally fed rats? Digestion. 1990;46 Suppl 2:177-81.
27. Morris GP, Beck PL, Herridge MS, Depew WT, Szewczuk MR, Wallace JL. Hapten-induced model of chronic inflammation and ulceration in the rat colon. Gastroenterology. 1989;96:795-803.

## Claims

1. A pharmaceutically active formulation of PYY or PYY agonist, wherein the PYY agonist is PYY[3-36], for use in the treatment of ulcerative colitis.

2. The formulation for use of claim 1, wherein the formulation reduces intestinal damage in the colon.

3. The formulation for use according to claim 1 or claim 2, in combination with a growth hormone or an anti-inflammatory agent.

4. The formulation for use according to any one of claims 1-3, wherein the PYY or PYY agonist is for administration by a route selected from the group consisting of intravenous, intraperitoneal, subcutaneous, intramuscular, oral, rectal, topical, transmucosal, or nasal administration, or pulmonary inhalation.

5. The formulation for use according to any one of claims 1-4, wherein the PYY or PYY agonist is for administration to a 50 kg patient in the amount of 100 µg to 500 mg/day.

6. The formulation for use of claim 1 in the form of a probiotic bacterium comprising a nucleic acid encoding the PYY or PYY agonist.

## Patentansprüche

1. Pharmazeutisch aktive Zubereitung von PYY oder eines PYY-Agonisten, wobei es sich bei dem PYY-Agonisten um PYY[3-36] handelt, zur Verwendung bei der Behandlung von Colitis ulcerosa.

2. Zubereitung zur Verwendung gemäß Anspruch 1, wobei die Zubereitung die Darmschädigung im Kolon reduziert.

3. Zubereitung zur Verwendung gemäß Anspruch 1 oder 2 in Kombination mit einem Wachstumshormon oder einem entzündungshemmenden Mittel.

4. Zubereitung zur Verwendung gemäß einem der Ansprüche 1 bis 3, wobei das PYY oder der PYY-Agonist zur Verabreichung auf einem Weg vorgesehen ist, der aus der Gruppe ausgewählt ist, die aus intravenöser, intraperitonealer, subkutaner, intramuskulärer, oraler, rektaler, topischer, transmukosaler oder nasaler Verabreichung oder Lungeninhalation besteht.

5. Zubereitung zur Verwendung gemäß einem der Ansprüche 1 bis 4, wobei das PYY oder der PYY-Agonist zur Verabreichung an einen 50-kg-Patienten in einer Menge von 100 µg bis 500 mg pro Tag vorgesehen ist.

6. Zubereitung zur Verwendung gemäß Anspruch 1 in Form eines probiotischen Bakteriums, das eine Nucleinsäure umfasst, die das PYY oder den PYY-Agonisten codiert.

## Revendications

1. Formulation pharmaceutiquement active de PYY ou d'agoniste de PYY, où l'agoniste de PYY est PYY[3-36], destinée à être utilisée dans le traitement de la colite ulcéreuse.

2. Formulation destinée à être utilisée selon la revendication 1 où la formulation réduit les lésions intestinales dans le côlon.

3. Formulation destinée à être utilisée selon la revendication 1 ou la revendication 2 en combinaison avec une hormone de croissance ou un agent anti-inflammatoire.

4. Formulation destinée à être utilisée selon l'une quelconque des revendications 1-3 où le PYY ou l'agoniste de PYY est destiné à l'administration par une voie choisie dans le groupe consistant en l'administration intraveineuse, intrapéritonéale, sous-cutanée, intramusculaire, orale, rectale, topique, transmuqueuse, ou nasale, ou l'inhalation pulmonaire.

5. Formulation destinée à être utilisée selon l'une quelconque des revendications 1-4 où le PYY ou l'agoniste de PYY est destiné à l'administration à un patient de 50 kg en la quantité de 100 µg à 500 mg/jour.

6. Formulation destinée à être utilisée selon la revendication 1 sous forme d'une bactérie probiotique comprenant un acide nucléique codant le PYY ou l'agoniste de PYY.


### SEQUENCE LISTING

<110> Amylin Pharmaceuticals, Inc.
<120> Prevention and/or Treatment of Inflammatory Bowel Disease Using PYY or Agonists Thereof
<130> 54061.8101.WO00
<150> 60/388,930
   <151> 2002-06-14
<160> 4
<170> PatentIn version 3.2
<210> 1
   <211> 36
   <212> PRT
   <213> Homo sapiens
<220>
<221> MISC_FEATURE
<223> Pancreatic polypeptide (PP)
<400> 1
   Ala Pro Leu Glu Pro Val Tyr Pro Gly Asp Asn Ala Thr Pro Glu Gln
   1 5 10 15
   Met Ala Gln Tyr Ala Ala Asp Leu Arg Arg Tyr Ile Asn Met Leu Thr
   20 25 30
   Arg Pro Arg Tyr
   35
<210> 2
   <211> 36
   <212> PRT
   <213> Homo sapiens
<220>
   <221> MISC_FEATURE
   <223> Peptide YY (PYY)
<400> 2
   Tyr Pro Ile Lys Pro Glu Ala Pro Gly Glu Asp Ala Ser Pro Glu Glu
   1 5 10 15
   Leu Asn Arg Tyr Tyr Ala Ser Leu Arg His Tyr Leu Asn Leu Val Thr
   20 25 30
   Arg Gln Arg Tyr
   35
<210> 3
   <211> 34
   <212> PRT
   <213> Homo sapiens
<220>
   <221> MISC_FEATURE
   <223> Peptide YY[3-36]
<400> 3
   Ile Lys Pro Glu Ala Pro Gly Glu Asp Ala Ser Pro Glu Glu Leu Asn
   1 5 10 15
   Arg Tyr Tyr Ala Ser Leu Arg His Tyr Leu Asn Leu Val Thr Arg Gln
   20 25 30
   Arg Tyr
<210> 4
   <211> 36
   <212> PRT
   <213> Homo sapiens
<220>
   <221> MISC_FEATURE
   <223> Neuropeptide Y (NPY)
<400> 4
   Tyr Pro Ser Lys Pro Asp Asn Pro Gly Glu Asp Ala Pro Ala Glu Asp
   1               5                   10                  15
   Met Ala Arg Tyr Tyr Ser Ala Leu Arg His Tyr Ile Asn Leu Ile Thr
               20                  25                  30
   Arg Gln Arg Tyr
           35